(19) Europäisches Patentamt
European Patent Office
Office européen des brevets

(11) Publication number: **0 400 190 A1**

# (12) EUROPEAN PATENT APPLICATION

(21) Application number: 89110014.1

(22) Date of filing: 02.06.89

(51) Int. Cl.5: C07D 295/08, A61K 31/33, A61K 31/145, C07C 229/24, C07C 229/26, C07C 279/14

(43) Date of publication of application: 05.12.90 Bulletin 90/49

(84) Designated Contracting States: BE DE ES FR GB GR LU NL

(71) Applicant: MAGIS FARMACEUTICI S.p.A.
Via Cacciamali 34/36/38
I-25125 Brescia(IT)

(72) Inventor: Puricelli, Laura
Via Taramelli, 7
I-25100 Brescia(IT)

(74) Representative: Gervasi, Gemma et al
NOTARBARTOLO & GERVASI Srl Viale Bianca Maria 33
I-20122 Milan(IT)

## (54) New water-soluble derivatives of nabumetone, a process for their preparation and pharmaceutical compositions which contain them.

(57) Water-soluble nabumetone enamine derivatives of formula 1 are described.

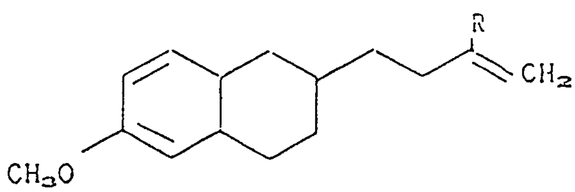

(I)

R represents an N-radical deriving from morpholine, pyrrolidine, piperidine, amino acid ester or amino acid sodium salt.

The obtained enamines hydrolyse in vitro in an acid environment and hydrolyse in vivo in plasma to regenerate nabumetone.

EP 0 400 190 A1

# NEW WATER-SOLUBLE DERIVATIVES OF NABUMETONE, A PROCESS FOR THEIR PREPARATION AND PHARMACEUTICAL COMPOSITIONS WHICH CONTAIN THEM

Nabumetone enamines are described of general formula (I):

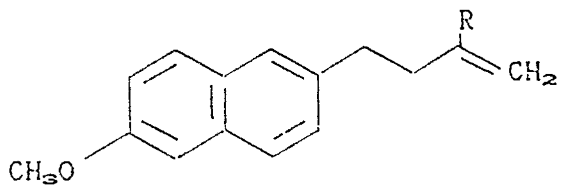

(I)

where R represents an N-radical deriving from morpholine, pyrrolidine, piperidine, amino acid ester or amino acid sodium salt.

The new compounds of general formula (I) possess interesting anti-inflammatory and analgesic activity while being free of damaging or undesirable side-effects on the cardiovascular system.

Compared with nabumetone, the new compounds have the characteristic of being water-soluble and can therefore be advantageously used in injectable forms.

The present invention therefore also relates to pharmaceutical compositions characterised by containing as active principle a therapeutically effective quantity of the compounds of general formula (I) (250 mg, 500 mg, 1000 mg or 1500 mg), either as such or associated with pharmaceutically acceptable carriers, diluents, solvents and/or excipients.

The present invention further relates to the process for preparing the compound of formula (I), characterised by reacting nabumetone with morpholine, pyrrolidine, piperidine or amino acid esters in benzene under hot conditions in the presence of p-toluenesulphonic acid in catalytic quantity and separating the water which forms; or by reacting nabumetone with an ester under cold conditions in the presence of titanium tetrachloride; or alternatively by reacting nabumetone with amino acid sodium salts in benzene and absolute ethyl alcohol, and separating the azeotrope.

The synthesis proceeds in accordance with the following general scheme:

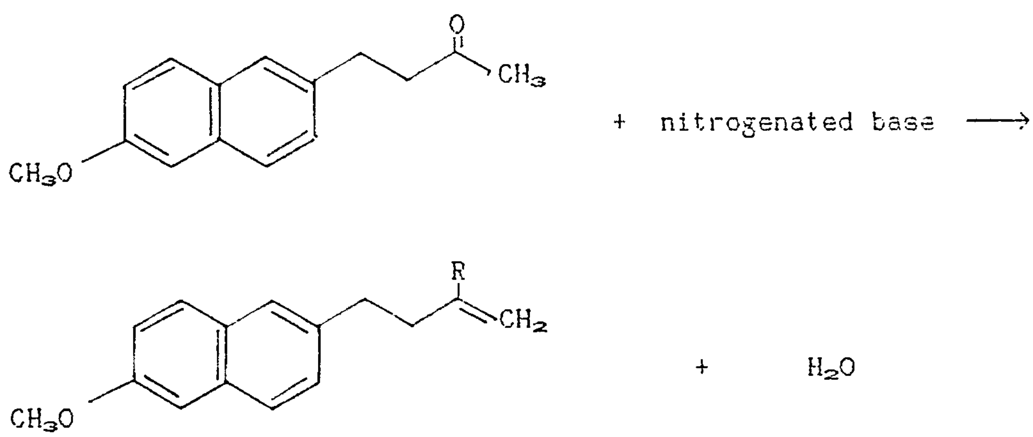


The following examples illustrate the invention but without limiting it.

EXAMPLE 1 morpholine/nabumetone

A mixture of 142.68 g (0.625 moles) of nabumetone, 81.0 g (0.930 moles) of morpholine, 200 mg of p-toluenesulphonic acid and 250 ml of benzene are fed into a 500 ml flask fitted with a water separator and reflux condenser protected by a trap containing calcium chloride.

The mixture is heated under reflux, the water is separated after 180 hours.

The mixture is cooled to ambient temperature, 200 mg of sodium ethoxide are added to remove the p-toluenesulphonic acid, and the mixture concentrated in a rotary evaporator (50 °C, 80-100 mm).

The crude oil obtained is distilled under reduced pressure in a flask fitted with a Vigreux column.

40-50 ml of a morpholine-nabumetone mixture separate at a temperature of between 40 and 90 °C (20 mm).

The residue consists of morpholine nabumetone (about 111 g).

Spectral analyses confirm the structure.

| Elementary analysis | C | H | N |
|---|---|---|---|
| calculated %<br>found %<br>M.W. 297.797 | 76.632<br>76.7 | 8.05<br>8.1 | 4.7035<br>4.68 |

EXAMPLE 2 L-glutamic acid dimethylester/nabumetone

About 180 g of L-glutamic acid dimethylester, 91.2 g (0.4 moles) of nabumetone dissolved in 200 ml of anhydrous ether and 220 ml of a 1 M solution of titanium tetrachloride in hexane are fed under a stream of nitrogen into a 1 litre flask fitted with a stirrer and placed in a cold acetone bath.

The solution is left for 2 hours in the cold bath while stirring, and then for 30 minutes at ambient temperature.

It is filtered through a Büchner funnel under a stream of nitrogen into a 1 litre flask.

The ether is eliminated under vacuum.

The residue of about 100 g is crystallised from ether and acetone.

Spectral analyses confirm the structure.

| Elementary analysis | C | H | N |
|---|---|---|---|
| calculated %<br>found %<br>M.W. 385 | 68.58<br>68.5 | 7<br>7.05 | 3.63<br>3.65 |

EXAMPLE 3

Using the procedures of Examples 1 and 2 the amines of pyrrolidine, of piperidine and of amino acid esters (glycerin, aspartic) are obtained.

EXAMPLE 4

48.525 g of the anhydrous sodium salt of glycine dissolved in 200 ml of absolute ethanol and 130 g of nabumetone dissolved in 600 ml of benzene are transferred into a flask fitted with a stirrer and an azeotrope separator. The mixture is heated under reflux for 5 hours while recycling the benzene, after which a further 200 ml of absolute ethanol are added and distillation is continued until ethanol is completely eliminated.

The mixture is filtered and the residue washed with ether and dried in an oven at 40 °C.

About 140 g of product are obtained.

Spectral analyses confirm the structure.

| Elementary analysis | C | H | N | Na |
|---|---|---|---|---|
| calculated % | 66.44 | 5.902 | 4.557 | 7.48 |
| found % | 66.5 | 5.95 | 4.5 | 7.45 |
| M.W. 307.327 | | | | |

EXAMPLE 5

Using the procedure of Example 4 the nabumetone enamines of the sodium salts of aspartic acid, glutamic acid, lysine, arginine and glutamine are obtained.

The pharmaco-biological characteristics of the nabumetone enamines according to the present invention are briefly reported.

Acute toxicity was studied in the mouse by the Weill method (Weill C.S. Biomed. J.8, 2491952).

The $LD_{50}$ for the nabumetone enamines was about 500 mg/kg.

The anti-inflammatory activity of these enamines was studied in comparison with acetylsalicylic acid on edema induced in the rat by formalin.

The $ED_{50}$ for the enamines was about 150 mg/kg whereas for acetylsalicylic acid it is about 313 mg/kg.

The analgesic activity was measured by the Writhing test using p-phenylquinone on mice treated orally with the nabumetone enamines in comparison with acetylsalicylic acid, the results showing an $ED_{50}$ (mg/Kg) for the enamines of about 80 whereas for acetylsalicylic acid the value is about 106.1.

The mean hydrolysis life of the enamines is reported in the following table:

| Mean life in phosphate buffer at pH 7.4 | | Mean life in buffer at pH 3.0 |
|---|---|---|
| with plasma | without plasma | |
| 30 hours | > 30 hours | 30 minutes |

**Claims**

1. Nabumetone enamines of general formula (I):

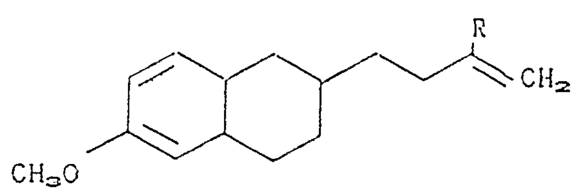

(I)

where R represents an N-radical deriving from morpholine, pyrrolidine, piperidine, amino acid ester or amino acid sodium salt.

2. A process for preparing compounds of formula (I), according to claim 1, characterised by reacting nabumetone under reflux with an excess of RH amine in the presence of a catalytic quantity of p-toluene-sulphonic acid in benzene, and continuously removing the water of reaction by azeotropic distillation.

3. A process for preparing compounds according to claim 1, characterised by reacting under cold conditions nabumetone dissolved in ether with an excess of RH amine in the presence of titanium chloride.

4. A process for preparing compounds according to claim 1, characterised by reacting under reflux in a flask fitted with an azeotrope separator nabumetone dissolved in benzene with amino acid sodium salt

dissolved in absolute ethanol.

5. Pharmaceutical compositions possessing anti-inflammatory and analgesic action comprising an effective quantity of the enamines claimed in claim 1.

6. Pharmaceutical compositions as claimed in claim 5, in the form of normal or delayed release tablets, pills, capsules, sachets, ready-made or extemporaneous syrup, suppositories or extemporaneous injectable forms.

Claims for the following Contracting States: GR, ES

1. A process for preparing nabumetone enamines of general formula (I):

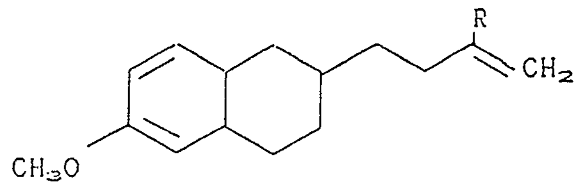

(I)

characterised by reacting nabumetone under reflux with an excess of RH amine in the presence of a catalytic quantity of p-toluene-sulphonic acid in benzene, and continuously removing the water of reaction by azeotropic distillation.

2. A process for preparing compounds of formula (I), characterised by reacting under cold conditions nabumetone dissolved in ether with an excess of RH amine in the presence of titanium chloride.

3. A process for preparing compounds of formula (I), characterised by reacting under reflux in a flask fitted with an azeotrope separator nabumetone dissolved in benzene with amino acid sodium salt dissolved in absolute ethanol.

European Patent Office

# EUROPEAN SEARCH REPORT

Application Number

EP 89 11 0014

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | J. MED. CHEM. vol. 21, no. 12, Washington D.C. pages 1260-1264; A.C. GOUDIE E.A.: "4-(6-Methoxy-2-naphthyl)butan-2-one and Related Analogues, a Novel Structural Class of Antiinflammatory Compounds" * the whole document * ----- | 1 | C 07 C 229/24<br>C 07 D 295/08<br>A 61 K 31/195<br>A 61 K 31/33<br>C 07 C 279/14 |
| | | | **TECHNICAL FIELDS SEARCHED (Int. Cl.5)** |
| | | | C 07 C 229/00<br>C 07 D 295/00 |

The present search report has been drawn up for all claims

| Place of search | Date of completion of the search | Examiner |
|---|---|---|
| THE HAGUE | 25-01-1990 | PAUWELS G.R.A. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)